# EUROPEAN PATENT SPECIFICATION

(11) **EP 3 381 409 B1**
(45) Date of publication and mention of the grant of the patent: **25.12.2019**
(21) Application number: 18158728.8
(22) Date of filing: 16.05.2012
(51) Int. Cl.: A61F 2/24, A61F 2/962

(54) **INVERSION DELIVERY DEVICE FOR A PROSTHESIS**
INVERSIONSAUSGABEVORRICHTUNG FÜR EINE PROTHESE
DISPOSITIF D'INVERSION POUR PROTHÈSE

(30) Priority: 16.05.2011 US 201161486682 P
(43) Date of publication of application: 03.10.2018
(62) Divisional of application: 12785404.0
(73) Proprietor: Hlt, Inc., Maple Grove, MN 55369 (US)
(72) Inventor: GAINOR, John P., Mendota Heights, MN 55118 (US); WILSON, Robert F., Roseville, MN 55113 (US); NELSON, Dale K., Inver Grove Heights, MN 55077 (US); CZYSCON, Joseph S, Plymouth, MN 55446 (US)
(74) Representative: KIPA AB

(56) References cited:
- WO-A1-2008/072838

## Description

### BACKGROUND OF THE INVENTION

There has been a significant movement toward developing and performing cardiovascular surgeries using a percutaneous approach. Through the use of one or more catheters that are introduced through, for example, the femoral artery, tools and devices can be delivered to a desired area in the cardiovascular system to perform many number of complicated procedures that normally otherwise require an invasive surgical procedure. Such approaches greatly reduce the trauma endured by the patient and can significantly reduce recovery periods. The percutaneous approach is particularly attractive as an alternative to performing open-heart surgery.

Valve replacement surgery provides one example of an area where percutaneous solutions are being developed. A number of diseases result in a thickening, and subsequent immobility or reduced mobility, of heart valve leaflets. Such immobility also may lead to a narrowing, or stenosis, of the passageway through the valve. The increased resistance to blood flow that a stenosed valve presents can eventually lead to heart failure and ultimately death.

Treating valve stenosis or regurgitation has heretofore involved complete removal of the existing native valve through an open-heart procedure followed by the implantation of a prosthetic valve. Naturally, this is a heavily invasive procedure and inflicts great trauma on the body leading usually to great discomfort and considerable recovery time. It is also a sophisticated procedure that requires great expertise and talent to perform.

Historically, such valve replacement surgery has been performed using traditional open-heart surgery where the chest is opened, the heart stopped, the patient placed on cardiopulmonary bypass, the native valve excised and the replacement valve attached. A proposed percutaneous valve replacement alternative method on the other hand, is disclosed in U.S. Pat. No. 6,168,614 issued to Andersen et al. In this patent, the prosthetic valve is mounted on a stent that is collapsed to a size that fits within a catheter. The catheter is then inserted into the patient's vasculature and moved so as to position the collapsed stent at the location of the native valve. A deployment mechanism is activated that expands the stent containing the replacement valve against the valve cusps. The expanded structure includes a stent configured to have a valve shape with valve leaflet supports begins to take on the function of the native valve. As a result, a full valve replacement has been achieved but at a significantly reduced physical impact to the patient.

However, this approach has decided shortcomings. One particular drawback with the percutaneous approach disclosed in the Andersen '614 patent is the difficulty in preventing leakage around the perimeter of the new valve after implantation. Since the tissue of the native valve remains within the lumen, there is a strong likelihood that the commissural junctions and fusion points of the valve tissue (as pushed apart and fixed by the stent) will make sealing around the prosthetic valve difficult. In practice, this has often led to severe leakage of blood around the stent apparatus.

Other drawbacks of the Andersen '614 approach pertain to its reliance on stents as support scaffolding for the prosthetic valve. First, stents can create emboli when they expand. Second, stents are typically not effective at trapping the emboli they dislodge, either during or after deployment. Third, stents do not typically conform to the features of the native lumen in which they are placed, making a prosthetic valve housed within a stent subject to paravalvular leakage. Fourth, stents are subject to a tradeoff between strength and compressibility. Fifth, stents cannot be retrieved once deployed. Sixth, stents have an inherent strength that is not adjustable.

As to the first drawback, stents usually fall into one of two categories: self-expanding stents and expandable stents. Self-expanding stents are compressed when loaded into a catheter and expand to their original, non-compressed size when released from the catheter. These are typically made of Nitinol. Balloon expandable stents are loaded into a catheter in a compressed but relaxed state. These are typically made from stainless steel or other malleable metals. A balloon is placed within the stent. Upon deployment, the catheter is retracted and the balloon inflated, thereby expanding the stent to a desired size. Both of these stent types exhibit significant force upon expansion. The force is usually strong enough to crack or pop thrombosis, thereby causing pieces of atherosclerotic plaque to dislodge and become emboli. If the stent is being implanted to treat a stenosed vessel, a certain degree of such expansion is desirable. However, if the stent is merely being implanted to displace native valves, less force may be desirable to reduce the chance of creating emboli.

As to the second drawback, if emboli are created, expanded stents usually have members that are too spaced apart to be effective to trap any dislodged material. Often, secondary precautions must be taken including the use of nets and irrigation ports.

The third drawback is due to the relative inflexibility of stents. Stents typically rely on the elastic nature of the native vessel to conform around the stent. Stents used to open a restricted vessel do not require a seal between the vessel and the stent. However, when using a stent to displace native valves and house a prosthetic valve, a seal between the stent and the vessel is necessary to prevent paravalvular leakage. Due to the non-conforming nature of stents, this seal is hard to achieve, especially when displacing stenosed valve leaflets.

The fourth drawback is the tradeoff between compressibility and strength. Stents are made stronger or larger by manufacturing them with thicker members. Stronger stents are thus not as compressible as weaker stents. Most stents suitable for use in a valve are not compressible enough to be placed in a thin catheter, such as a 14Fr catheter. Larger delivery catheters are more difficult to maneuver to a target area and also result in more trauma to the patient.

The fifth drawback of stents is that they are not easily retrievable. Once deployed, a stent may not be recompressed and drawn back into the catheter for repositioning due to the non-elastic deformation (stainless steel) or the radial force required to maintain the stent in place (Nitinol). Thus, if a physician is unsatisfied with the deployed location or orientation of a stent, there is little he or she can do to correct the problem.

The sixth drawback listed above is that stents have an inherent strength and are thus not adjustable. As previously stated, stronger stents are made with stronger members. Once a stent is selected and deployed, there is little a physician can do if the stent proves to be too strong or too weak.

Various embodiments of devices that solve these problems are introduced in U.S. Patent Publication No. 2006/0271166 to Thill et al., entitled "Stentless Support Structure," the contents of which are incorporated herein in their entirety. This publication teaches a braided mesh tube that is capable of folding back and forth into itself to build, in situ, a support structure that is strong enough to hold back the leaflets of a native valve sufficiently to successfully deploy a replacement valve, thus obviating the need for excision of the native valve. Advantageously, because of the inverting nature of these devices, the braided mesh, in an elongated delivery configuration, does not need to possess the strength to accomplish native valve displacement until the inversion process occurs. This allows the mesh tube to be constructed such that, in the elongated delivery state, the tube can be compressed into a very small catheter, such as a 14Fr or smaller catheter. Such a small catheter significantly reduces patient trauma and allows for easy percutaneous, intraluminal navigation through the blood vessels. It is to be understood that terms like transluminal and percutaneous, as used herein, are expressly defined as navigation to a target location through and axially along the lumen of a blood vessel or blood vessels as opposed to surgically cutting the target vessel or heart open and installing the device manually. It is further to be understood that the term "mesh" as used herein describes a material constructed of one or more braided or woven strands.

In order to accomplish the folding back and forth feature of this device, there are preformed, circumferential folds in the device. One embodiment has two circumferential folds that are longitudinally spaced apart in the extended configuration. One of these folds is preformed to fold inwardly, and the other is preformed to fold outwardly. These preformed folds, when released out of a catheter, tend to return to a folded configuration that has a z-like cross-section. This cross-section design results not only because the inward pre-formed fold folds inwardly and the outward pre-formed fold folds outwardly, but because these folds reverse longitudinal positions once folded. If the inward preformed fold is distal of the outward preformed fold in the extended position, in the folded position the inward preformed fold will be proximal of the outward preformed fold. This design allows a valve on a distal end of the device to be drawn into the device when folded, without requiring the valve itself to be inverted or everted. In one embodiment having two preformed folds, the inversion process thus results in a three-layered configuration that could be significantly shorter than the extended length, depending on the spacing of the folds.

In the development of the devices described in the aforementioned publication, U.S. Pat. Pub. 2006/0271166, it was found that, occasionally, it was advantageous to use an additional device to hold the outermost layer of the implant axially in place while inversion of a layer was being effected. This gave rise to the delivery tool shown and described in U.S. Patent Publication 2008/0082165 to Wilson et al., entitled "Delivery Tool For Percutaneous Delivery Of A Prosthesis." This delivery tool includes an expandable mesh region that, when axially compressed, flares outwardly to form a bulbous or rounded structure of increased radius. Further axial compression creates a flat, disc-like surface. In use, the device is extended through an implant prior to releasing the implant from the delivery catheter. The device is then expanded to the disc-like configuration and pulled proximally to act as a backstop at a desired target location, against which the implant is delivered. Thus, the disc-like device prevents axial migration of the implant in a distal direction if and when distal force is placed on the implant during inversion of the second or subsequent layers into the first layer.

WO 2008/072838 discloses an inserting device of an artificial blood stent.

It has been found, however, that in some cases, depending on target location and patient anatomy, there is insufficient space in a distal axial direction beyond the target location to efficiently use this delivery device. For example, some patients may have limited left ventricular space, which may prohibit the use of the backstop device.

There is thus a need for a device that is able to prevent axial migration of the aforementioned braided implant devices during inversion, but does not require significant space distally beyond the target location.

### BRIEF SUMMARY OF THE INVENTION

The invention is defined by the claims.

Embodiments provide a delivery device that holds a braided implant in a desired location during inversion of a subsequent layer into a first layer. More specifically, embodiments provides a delivery device that releasably attaches at or near a first fold location, hereinafter referred to as the "aortic flare," which is a hinge point around which the inversion of the implant used with the present invention occurs.

Through attachment to the aortic flare, the delivery device enables precise positioning and inversion by limiting advancement of a portion of the implant while continuing to advance the remainder of the device. Hence, inversion is effected at a location selected by the user, independent of patient anatomy or geometric interference.

Two aspects of the present invention provide reliable performance of the delivery device. A first aspect is an attachment mechanism that can be mounted to a braided device without requiring significant modification to the function of the braided device. This attachment mechanism provides device stabilization during the support structure inversion process. This attachment mechanism provides both attachment to the device and a release capability in some embodiments. A second aspect includes positioning mechanisms that prevent movement of the device in the target location during the inversion process.

Another aspect provides freedom of motion to the support structure anchors as the device is being deployed, but automatically actuates anchor locking mechanisms when the device has been advanced to the appropriate position for the inversion process.

### BRIEF DESCRIPTION OF THE DRAWINGS

Figure 1 is a partial cross-sectional view of an embodiment of a delivery device of the present invention with an implant loaded in a distal end thereof;
Figure 2 is a perspective view of a distal end of an embodiment of a pusher catheter of the present invention;
Figure 3 is a perspective view of an embodiment of a distal end of a release mechanism of the present invention in an open configuration;
Figure 4 is a perspective view of the mechanism of Figure 3 in a closed configuration;
Figure 5 is a plan cross-sectional view of an embodiment of a delivery device of the present invention, just prior to an inversion process of an implant;
Figure 6 is a plan cross-sectional view of an embodiment of a delivery device as shown in Figure 1, just after the implant has been inverted;
Figure 7-10 are perspective views of a pusher catheter of an embodiment of a delivery device;
Figure 11 is a side view of an embodiment of a delivery device of the present invention in a vessel of a patient;
Figure 12 is a side view of an embodiment of a delivery device as shown in Figure 11, just after crossing a heart valve;
Figure 13 is a side view of an embodiment of a delivery device as shown in Figure 11, in which an implant is partially deployed;
Figure 14 is a side view of an embodiment of a delivery device as shown in Figure 11, in which the tethers are tightened;
Figure 15 is a side view of an embodiment of a delivery device as shown in Figure 11, just after the implant has been inverted;
Figure 16 is a side view of an embodiment of a delivery device as shown in Figure 11, just after releasing and withdrawing the tethers from the implant;
Figure 17 is a side view of an embodiment of a delivery device as shown in Figure 11, just prior to releasing the attachment cables; and,
Figure 18 is a side view of an embodiment of the implant, just after release of the attachment cables.

### DETAILED DESCRIPTION OF THE INVENTION

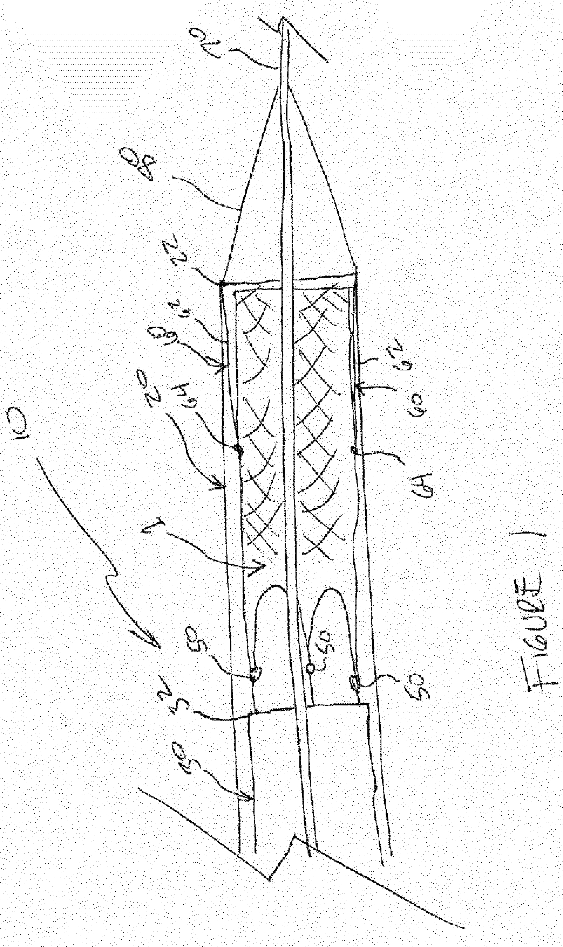

Referring now to the Figures and first to Figure 1, there is shown a delivery device 10 of the present invention. The delivery device generally includes a delivery catheter 20, and a pusher catheter 30 slidably contained within the delivery catheter 20. The pusher catheter 30 is preferably a multi-lumen catheter containing lumens for slidably containing and maintaining alignment of three attachment cables 40, each of which has a releasable grasping mechanism 50 at a distal end thereof. The delivery device 10 also includes at least one positioning mechanism 60 used to aid the tool or implant 1 in achieving a folded, deployed configuration from an extended, unfolded, navigation configuration. In one embodiment, the at least one positioning mechanism 60 is attached to a distal end of the delivery catheter 20. In another embodiment, the at least one positioning mechanism 60 is slidably contained within the delivery catheter 20, similar to the attachment cables 40.

The delivery catheter 20 is an external sheath defining a single lumen for housing the pusher catheter 30, the tool or implant 1, the attachment cables 40, and the positioning mechanisms 60. The delivery catheter 20, when loaded, houses a tool or implant 1 near its distal end 22. The implant 1 is preferably an implant similar to those taught and described in U.S. Patent Publication No. 2006/0271166.

The pusher catheter 30 may include up to seven lumens. Three lumens slidably house the three attachment cables 40. In an embodiment used over-the-wire, a fourth lumen accommodates a guidewire. In yet another embodiment, three additional lumens slidably house three positioning mechanisms, described below.

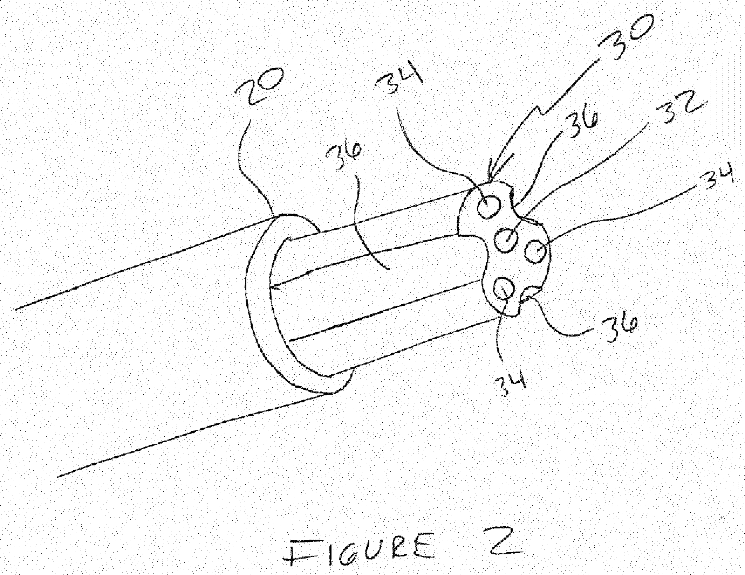

Figure 2 shows an embodiment of the pusher catheter 30 of the present invention defining seven lumens. The pusher catheter 30 includes a central guidewire lumen 32, and three lumens 34 that contain either the attachment cables 40 or the positioning mechanisms described below. The remaining three lumens 36 house the remaining attachment cables or positioning mechanisms. In order to save on space, the lumens 36 may be formed as external indentations, thereby relying on the inner wall of the delivery catheter 20 to complete the lumen and contain the remaining attachment cables or positioning mechanisms. In a preferred embodiment, the lumens 34 contain the attachment cables 40 and the lumens 36 contain the positioning mechanisms 60. In this embodiment, the pusher catheter 30 may continue to be advanced even if the positioning mechanisms 60 can no longer be advanced.

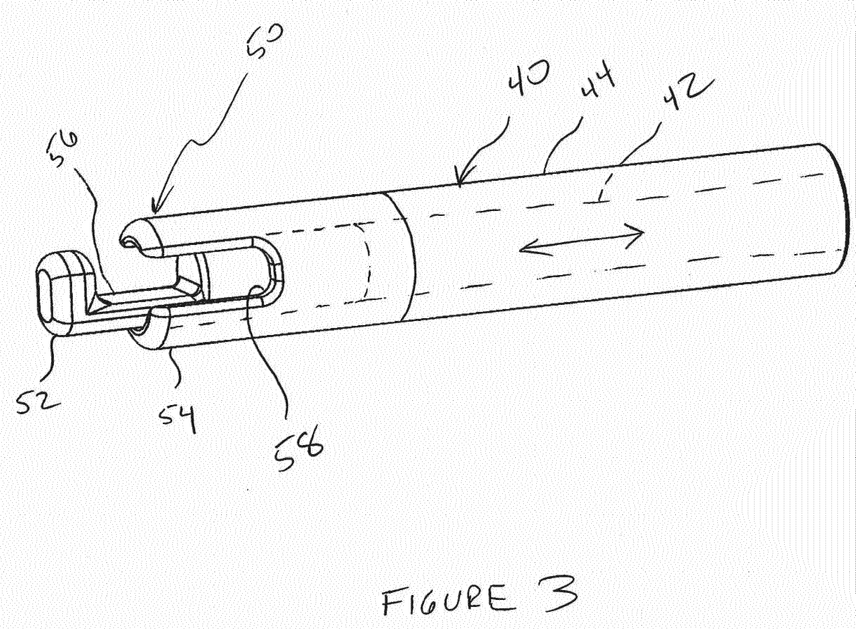

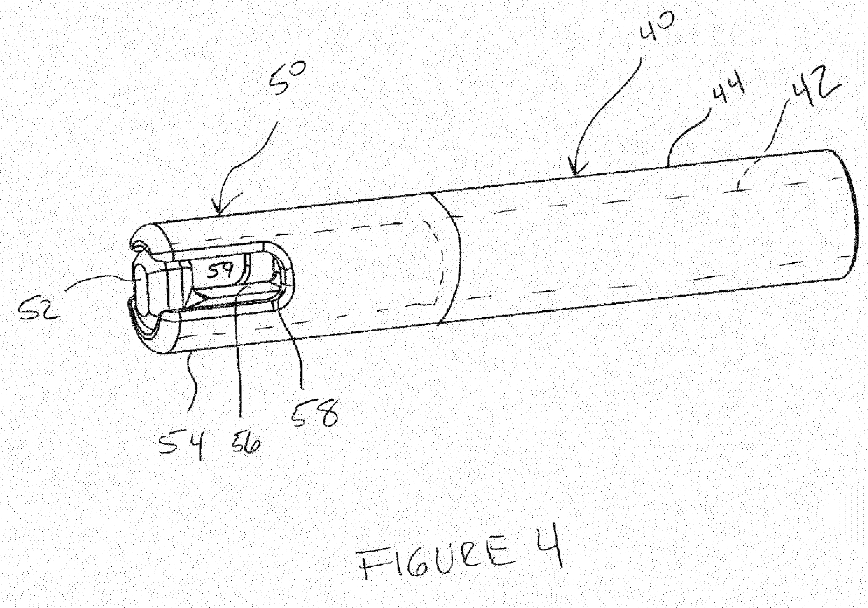

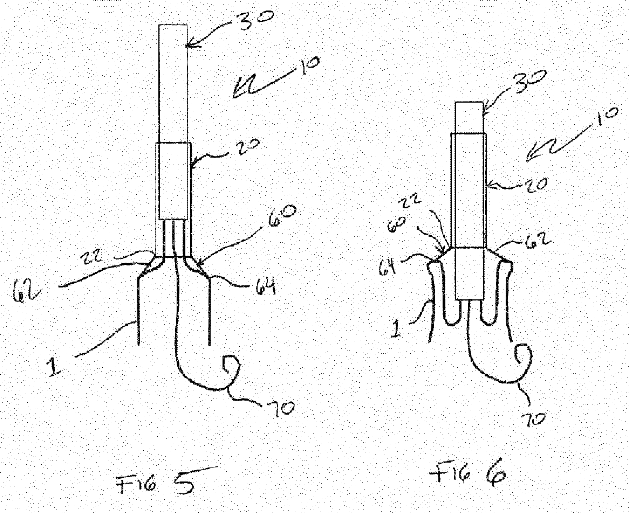

The releasable grasping mechanisms 50 may be similar to those shown and described in U.S. Pat. Pub. 2008/0082165 (at Figs. 5-8). Another embodiment of releasable grasping mechanisms is shown in Figures 3 and 4. The grasping mechanisms 50 are attached to commissural points on an implant 1 when the device 10 is loaded. The grasping mechanism 50 provide the ability to retract the implant back into the device 10 in the event that the physician feels doing so is appropriate.

Figure 3 shows a grasping mechanism 50 in an open configuration. The grasping mechanism 50 includes a hook 52 that slides within a mouth 54. The hook 52 defines a recess 56 sized to accommodate a component, such as a commissural point or a braid, of the tool or implant 1. The mouth 54 defines a slot 58 that is also sized to accommodate the component. Figure 4 shows that when the grasping mechanism 50 is in a closed configuration, the recess 56 and the slot 58 together define a passage 59 that traps the component therein.

The grasping mechanism 50 is attached to a distal end of an attachment cable 40. The attachment cable 40 includes a wire 42 attached to the hook 52 and an elastomeric sheath 44 that is attached to the mouth 54. The wire 42 and the hook 52 are slidably contained within the sheath 44 and the mouth 54. The sheath 44 is elastomeric such that it is capable of being compressed longitudinally. This feature prevents accidental release of a tool or component contained within the passage 59. For example, when pulling a tool or implant back into the delivery sheath 20 during a retrieval, a load is placed on the wire 42, causing the wire to stretch. If the sheath 44 were not compressed, the wire 42 could stretch enough to cause the hook 52 to exit the mouth 54, thereby assuming the open configuration of Figure 3. However, because the sheath 44 is compressed when the hook 52 is drawn into the mouth 54 during closing, the sheath 44 expands when the wire 42 is stretched, thereby maintaining the closed configuration of Figure 4.

The positioning mechanisms 60 aid in inverting the tool or component 1. In one embodiment, shown in Figures 1, 5 and 6, the positioning mechanisms 60 connect the distal end 22 of the delivery catheter 20 with a first inversion pre-fold point (also referred to herein as an "aortic flare") on the implant 1.

The positioning mechanisms 60 may comprise a plurality of tethers 62 and connectors 64. The tethers 62 may be any resilient strand-like material, flexible enough to invert from a navigation configuration to a deployment configuration. In the navigation configuration, as shown in Figure 1, the tethers extend proximally from the distal end of the delivery catheter 20, to the connectors 64. In the deployment configuration, shown in Figures 5 and 6, the tethers 62 extend distally from the distal end of the delivery catheter 20 to the connectors 64. In one embodiment, the connectors 64 are able to grasp any individual braid or strand of an implant 1. In another embodiment, the connectors 64 are designed to grasp the intersection of two braids or strands. In yet another embodiment, the connectors 64 are able to grasp discrete attachment points (e.g. wire loops, sutures, etc.), that have been integrated into the mesh implant or tool 1. The length of the tethers 62 are at least the length of the material of the implant 1 that extends distally of the connectors 64 when the implant 1 is loaded into the delivery catheter 20. This way, the implant 1 remains completely within the delivery catheter 20 in the navigation configuration.

In another embodiment, the positioning mechanisms 60 are similar in construction to the attachment cables 40 and releasable grasping mechanism 50. However, because the strength requirements of the positioning mechanisms 60 are less than those of the attachment cables 40 and their releasable grasping mechanisms 50, the positioning mechanisms 60 may be smaller in diameter, thereby allowing a smaller overall delivery device 10. Rather than being attached to the distal end of the delivery catheter 20, as described above, the positioning mechanisms 60 of this embodiment are slidably contained within the lumens 36 of the pusher catheter 30 shown in Figure 2.

Referring to Figures 5 and 6, the device 10 is designed to be able to pass over a guidewire 70 during navigation. A conical dilator 80 abuts against the distal end 22 of the delivery catheter 20 and is flush therewith. The conical dilator 80 allows the device 10 to be passed through the vasculature with minimal trauma. The conical dilator 80 is not physically attached to the delivery catheter 20, such that it is easily moved distally during delivery of the implant 1 to avoid interference with the deployment of the implant 1.

Having described the various components of the present invention, the various steps and configurations that occur during navigation and deployment of an implant can now be explained. Figure 1 shows the navigation configuration of the device 10. In the navigation configuration, the implant 1 is loaded into the distal end of the delivery catheter 20 such that the implant 1 is in an elongated, non-folded state. The pusher catheter 30 is positioned within the delivery catheter 20 with its distal end 22 proximal of the implant 1. The attachment cables 40 extend distally from the pusher catheter 30 and are connected to commissural points of the implant 1 with the releasable grasping mechanisms 50. The conical dilator 80 abuts against the distal end 22 of the delivery catheter 20. During navigation, the entire device 10 and implant 1 travel over a guidewire 70 to the target location.

Figure 5 shows the initial stages of deployment of the implant 1. The target location has been reached and the pusher catheter 30 is advanced through the delivery catheter 20 while the delivery catheter 20 remains stationary relative to the target location. Advancing the pusher catheter 30 causes the pusher catheter 30 to push the implant 1 out of the distal end 22 of the delivery catheter. As the implant 1 exits the delivery catheter 20 the implant 1 expands and the positioning mechanisms 60 are advanced through the delivery catheter 20 until the tethers 62 become taut, or in the case of the positioning mechanisms that are slidably contained within the lumens of the pusher catheter 30, the positioning mechanisms 60 can no longer be advanced.

As seen in Figure 6, further advancement of the pusher catheter 30 causes implant material that is proximal of the connectors 64 to invert into the implant material that is distal of the connector 64. This is because the positioning mechanisms 60 are taut and do not allow further distal advancement of the implant 1. As such, the inversion of the implant 1 is urged by the preformed fold in the implant, the expansion of the memory metal making up the implant 1, and the restraint provided by the positioning mechanisms 60. Notably, the transition of the implant from initial advancement to inversion happens automatically and is dictated by the length of the tethers 64. As such, operator experience is not required to initiate inversion. Nor is there any reliance on anatomical structure to provide friction against the implant to initiate inversion.

Once the implant 1 has been fully deployed, the implant 1 is fully functional prior to release. This allows verification of proper operation of the implant 1 via one or more imaging modalities prior to full release of the implant 1. If proper operation is not achieved, the grasping mechanisms 50 can be used to pull the implant 1 back into the delivery catheter 20 such that the implant may be either removed or redeployed. If proper operation is verified, the connectors 64 are actuated to release the braids or strands of the implant 1. The pusher catheter 30 and the delivery catheter 20 are withdrawn slightly while maintaining connection with the implant 1 and the device 10 via the releasable grasping mechanism. Subsequently, the grasping mechanisms 50 are actuated to release the commissural points of the implant 1. The pusher catheter 30 is retracted into the delivery catheter 20 and the delivery catheter 20 and the guidewire 70 are withdrawn from the patient.

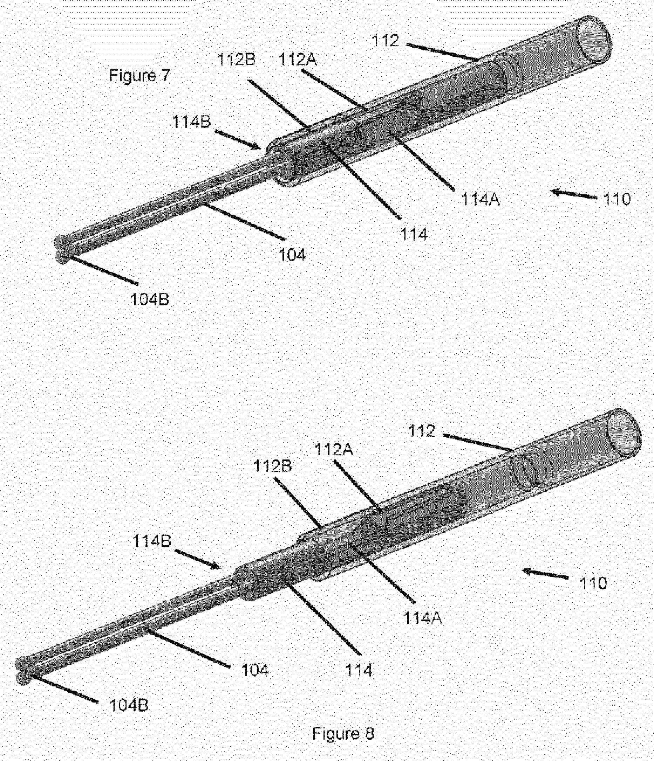

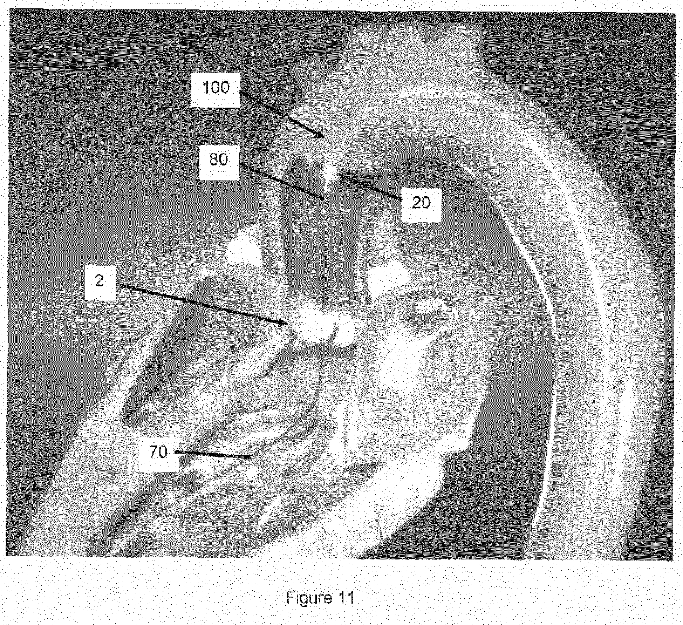

Figures 11-18 illustrate another embodiment of a delivery device 100 that is generally similar to the previously described delivery device 10, especially where noted with similar element numbers. However, the delivery device 100 includes a pusher catheter 110, seen best in Figures 7-10, having a sliding release mechanism for releasing a connection to the implant 1.

More specifically, the pusher catheter 110 includes a plurality of tethers 104 that are each arranged in a generally closed loop. These looped tethers 104 pass through portions of the implant 1 and therefore can maintain the implant 1 in a desired position during a procedure (e.g., can prevent distal movement of the implant 1). The tethers can be disconnected from the implant 1 by releasing one end of each of the tethers 104, effectively opening the loop shape. In this respect, withdrawal of the pusher catheter 110 also pulls the tethers 104 out of and away from the implant 1.

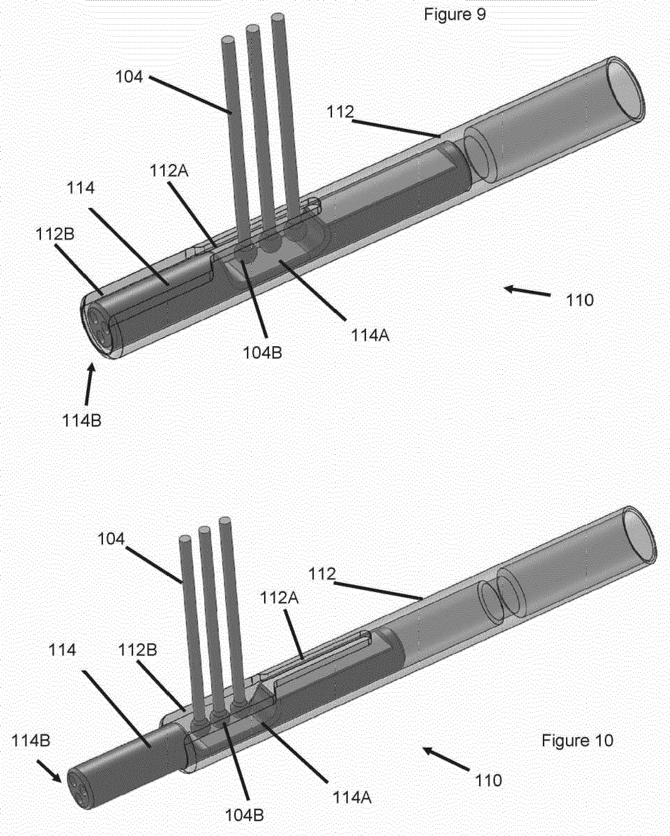

The release mechanism of the pusher catheter 110 is triggered by advancing a sliding member 114 to the position seen in Figures 8 and 10 from the retracted position seen in Figures 7 and 9. Note that the tethers 104 are connected to a distal end 114B of the pusher member 114 (e.g., either fixed in place or pass through the member 114 back to the proximal end of the catheter 110), but for illustrative purposes are not shown as such in Figures 9 and 10. Initially, the free ends 104B of the tethers 104 are located within a depression 114A of the sliding member 114 and are captured by a first slot 112A of an outer pusher sheath 112. When the sliding member 114 is advanced, the depression 114A is positioned beneath a wider, second slot 112B, which allows the free ends 104B of the tethers 104 to be released.

As best seen in Figure 9, the free ends 104B of the tethers 104 have a generally larger size or diameter than the remaining portion of the tether 104 and can have a variety of different shapes, such as rounded, spherical or even square. The first slot 112A has a width that is large enough to accommodate the diameter of the tether 104 but is smaller than the diameter of the free ends 104B, thereby allowing the tether 104 to laterally slide within the slot 112A without the free ends 104B from traversing through.

The second slot 112B is positioned distal to the first slot 112A and has a width that is larger than the free ends 104B. In this respect, once the depression 114A aligns under this second slot 112B, as seen in Figure 10, the free ends 104B are released, thereby allowing the tethers 104 to assume a generall linear configuration, similar to that in Figure 8.

While two slots are shown, a single slot may alternately be used in another embodiment. Specifically, the single slot may be similar in size to slot 112A, but extends to the distal end of the pusher sheath 112. In this respect, the free ends 104B are released when the depression 114A is advanced outside of the pusher sheath 112.

Although not shown in Figures 7-10, the pusher catheter 110 also includes lumens 36 and 32, similar to those seen in Figure 2. As with the previously described device 10, these lumens allow attachment cables 50 and a guidewire 70, respectively, to be used during a procedure.

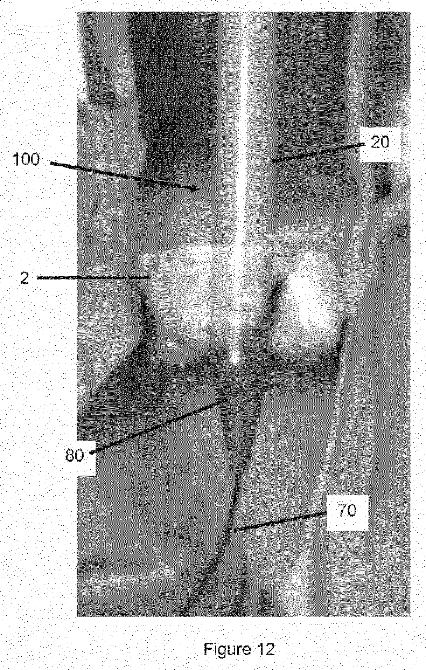

Figures 11-18 illustrate how the delivery device 100 can be used to deliver an implant 1 according to the present invention. As seen in Figure 11, a guidewire 70 is navigated across a native heart valve 4 of a patient and the delivery device 100 is advanced over the guidewire 70 until the conical dilator 80 passes through the native valve 4 (seen best in Figure 12).

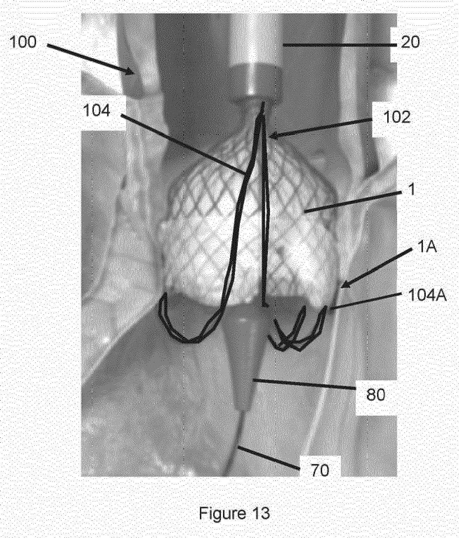

Turning to Figure 13, the delivery sheath 20 is proximally retracted to expose a portion of the implant 1 and the tethers 104. As the implant 1 becomes exposed, it self-expands outward against the native valve 4.

Each of the tethers 104 extend from the distal end 114B of the sliding member 114, pass through a distal region 1A of the implant 1 (e.g., through a distal loop or aperture), and then connect to the sliding release mechanism described in Figures 7-10. In other words, at area 104A, the tether 104 loops over a portion of the distal region 1A. In one embodiment, the tethers 104 are fixed from movement at the distal end 114B. In another embodiment, the tethers 104 continue through the distal end 114B to the proximal end of the pusher catheter 110, allowing the user to increase or decrease their tension, as needed.

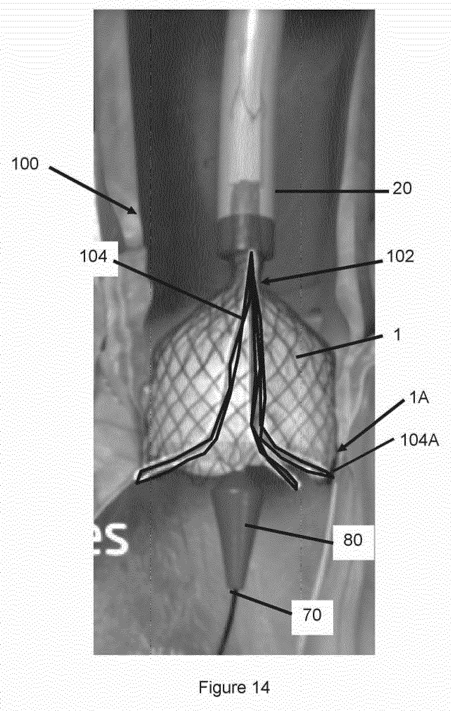

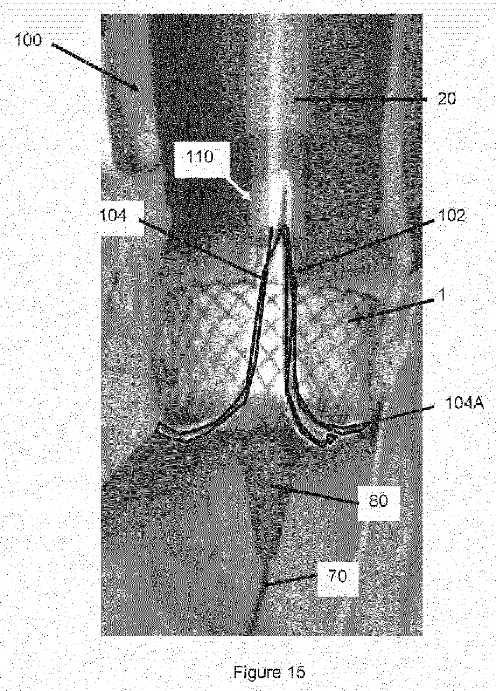

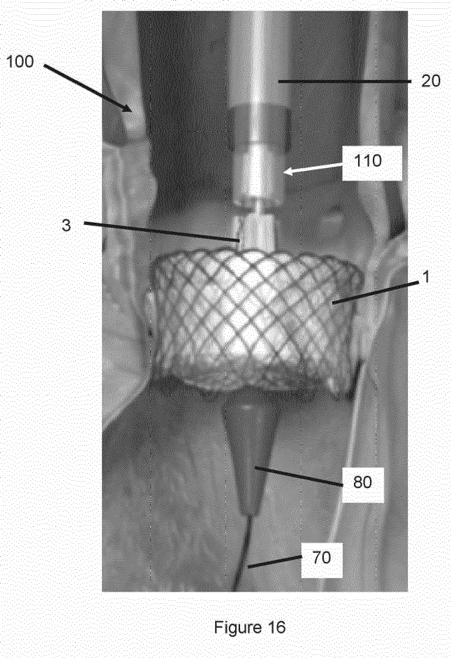

Next, the implant 1 is folded or inverted on itself by restraining the implant with the tethers 104 and pushing a proximal portion of the implant in a distal direction with the pusher catheter 110. Referring now to Figure 14, the tethers 104 are first tightened (i.e., pulled proximately) and kept taut as the pusher catheter 110 is advanced distally. As seen in Figure 15, this pushing and pulling force causes the implant 1 to fold along a preformed fold line. If the physician believes that the implant has been properly placed and folded, the tethers 104 can be disconnected from the implant 1 by releasing the free ends 104B from the pusher catheter 110, as previously described, and proximally pulling the tethers 104 into the pusher catheter 110 (Figure 16).

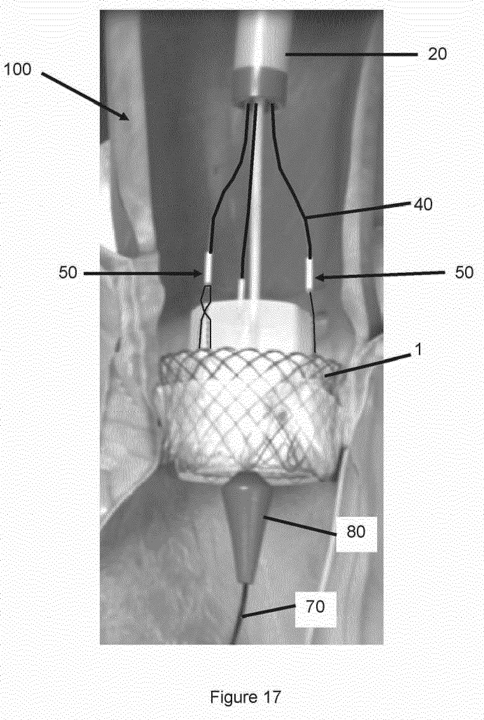

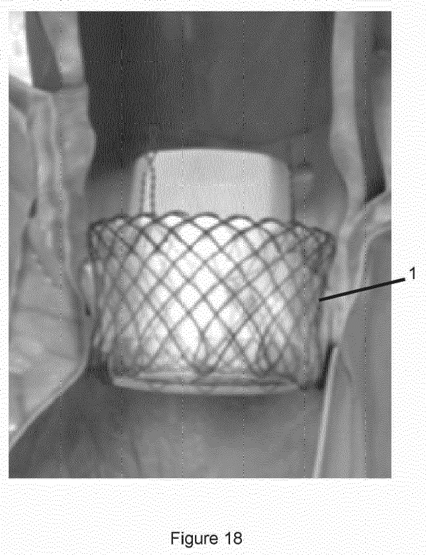

Once the tethers 104 have been removed, only the attachment cables 40 remain connected to the implant 1, as seen in Figure 17. As previously discussed, these cables 40 are attached to a proximal feature on the implant (e.g., the commissural points) and allow the physician to completely retract the implant 1 back into the delivery device 100 if a problem arises during the delivery procedure. If the physician is satisfied with the placement of the implant, the cables 40 are released by opening grasping mechanisms 50. Finally, the delivery device 100 and guidewire 70 is removed from the patient, leaving only the functioning valve implant 1, as seen in Figure 18.

Although the invention has been described in terms of particular embodiments and applications, one of ordinary skill in the art, in light of this teaching, can generate additional embodiments and modifications without departing from or exceeding the scope of the claimed invention, which is defined by the claims. Accordingly, it is to be understood that the drawings and descriptions herein are proffered by way of example to facilitate comprehension of the invention defined by the claims.

## Claims

1. A system for replacing a native heart valve with an invertible device comprising:
a delivery catheter (20);
a pusher catheter (30) slidably disposed within said delivery catheter (20);
a positioning mechanism (60) releasably connecting a device to said delivery catheter (20); and
a plurality of attachment cables (40) that are releasably connected to a proximal end of said device,
**characterized in that**
said device is an invertible device, and said positioning mechanism (60) is configured to limit distal movement of a distal portion of said invertible device during an implant inversion procedure in which a proximal portion of said invertible device folds into said distal portion.

2. The device of claim 1, wherein said positioning mechanism (60) comprises at least one tether (104) that is connected near a distal end (114B) of said invertible device and to said delivery catheter (20).

3. The device of claim 1, wherein said positioning mechanism comprises at least one tether (114B) that is connected to said invertible device and to said delivery catheter (20).

4. The device of claim 1, wherein said positioning mechanism (60) further comprises:
an outer sheath (112) having a first slot (112A); and,
a sliding member (114) located within said outer sheath (112) and having a depression (114A).

5. The device of claim 4 wherein said positioning mechanism comprises at least one tether (114B) having an end sized to fit within said depression (114A) of said sliding member (114).

## Patentansprüche

1. System, das das Ersetzen einer nativen Herzklappe mit einer kippbaren Vorrichtung gestattet, bestehend aus:
einem Einführkatheter (20),
einem Schubkatheter (30), der gleitbar im Inneren des vorerwähnten Einführkatheters (20) angeordnet ist,
einem Positioniermechanismus (60), der eine Vorrichtung freigebbar mit dem vorerwähnten Einführkatheter (20) verbindet,
einer Mehrzahl von Befestigungskabeln (40), die freigebbar an einem proximalen Ende der vorerwähnten Vorrichtung befestigt sind,
**dadurch gekennzeichnet, dass**
die vorerwähnte Vorrichtung kippbar ist und der vorerwähnte Positioniermechanismus (60) so ausgelegt ist, dass er die distale Bewegung eines distalen Bereichs der vorerwähnten kippbaren Vorrichtung während eines Kippvorgangs eines Implantats, bei dem ein proximaler Bereich der vorerwähnten kippbaren Vorrichtung im vorerwähnten distalen Bereich zusammengelegt wird, begrenzt.

2. Vorrichtung gemäß Anspruch 1, bei der der vorerwähnte Positioniermechanismus (60) mindestens einen Zahn (104) aufweist, der in der Nähe eines distalen Endes (114B) der vorerwähnten kippbaren Vorrichtung und mit dem vorerwähnten Einführkatheter (20) verbunden ist.

3. Vorrichtung gemäß Anspruch 1, bei der der vorerwähnte Positioniermechanismus mindestens einen Zahn (114B) aufweist, der mit der vorerwähnten kippbaren Vorrichtung und mit dem vorerwähnten Einführkatheter (20) verbunden ist.

4. Vorrichtung gemäß Anspruch 1, bei der der vorerwähnte Positioniermechanismus (60) weiterhin Folgendes aufweist:
eine äußere Hülle (112) mit einem ersten Schlitz (112A) und
ein Schiebeelement (114), das im Inneren der vorerwähnten äußeren Hülle (112) angebracht ist und eine Vertiefung (114A) aufweist.

5. Vorrichtung gemäß Anspruch 4, bei der der vorerwähnte Positioniermechanismus mindestens einen Zahn (114B) mit einem Ende aufweist, das so dimensioniert ist, dass es sich in die vorerwähnte Vertiefung (114A) des vorerwähnten Schiebeelements (114) legt.

## Revendications

1. Système permettant de remplacer une valve cardiaque native à l'aide d'un dispositif inversable comprenant :
un cathéter de pose (20) ;
un cathéter pousseur (30) disposé avec possibilité de coulissement à l'intérieur dudit cathéter de pose (20) ;
un mécanisme de positionnement (60) reliant de manière libérable un dispositif audit cathéter de pose (20) ; et
une pluralité de câbles d'attache (40) qui sont reliés de manière libérable à une extrémité proximale dudit dispositif,
**caractérisé en ce que**
ledit dispositif est un dispositif inversable, et ledit mécanisme de positionnement (60) est configuré pour limiter un déplacement distal d'une partie distale dudit dispositif inversable pendant une procédure d'inversion d'implant lors de laquelle une partie proximale dudit dispositif inversable se plie dans ladite partie distale.

2. Dispositif selon la revendication 1, dans lequel ledit mécanisme de positionnement (60) comprend au moins une dent (104) qui est reliée près d'une extrémité distale (114B) dudit dispositif inversable et audit cathéter de pose (20).

3. Dispositif selon la revendication 1, dans lequel ledit mécanisme de positionnement comprend au moins une dent (114B) qui est reliée audit dispositif inversable et audit cathéter de pose (20).

4. Dispositif selon la revendication 1, dans lequel ledit mécanisme de positionnement (60) comprend en outre :
une gaine extérieure (112) présentant une première fente (112A) ; et
un élément coulissant (114) positionné à l'intérieur de ladite gaine extérieure (112) et présentant un creux (114A).

5. Dispositif selon la revendication 4, dans lequel ledit mécanisme de positionnement comprend au moins une dent (114B) ayant une extrémité dimensionnée pour loger à l'intérieur dudit creux (114A) dudit élément coulissant (114).
